# EUROPEAN PATENT APPLICATION

(11) **EP 3 995 161 A1**
(43) Date of publication of application: **11.05.2022**
(21) Application number: 21206370.5
(22) Date of filing: 04.11.2021
(51) Int. Cl.: A61M 1/00, A61M 3/02, A61M 39/22, A61M 39/24

(54) **HAND ACTUATED SUCTION AND IRRIGATION DEVICE**

(30) Priority: 05.11.2020 US 202063109945 P; 21.09.2021 US 202117481143
(71) Applicant: Covidien LP, Mansfield, MA 02048 (US)
(72) Inventor: LOBO, Astley C., West Haven, CT Connecticut 06516 (US); DESJARDIN, Kevin, Prospect, CT Connecticut 06712 (US)
(74) Representative: Maschio, Antonio

(57) **Abstract**

A hand actuated suction and irrigation device enables the clinician to selectively provide suction or irrigation at a surgical site. In particular, the suction and irrigation device enables the clinician to control the amount of suction or irrigation provided at the surgical site through an ergonomically designed handle assembly. A clinician may selectively choose the suction or irrigation setting on the device by manipulating the first and second valve assemblies on opposing sides of a pump.

## Description

### CROSS-REFERENCE TO RELATED APPLICATIONS

This application claims the benefit of, and priority to, U.S. Provisional Patent Application No. 63/109,945 filed on November 5, 2020. The entire contents of each of these applications is hereby incorporated herein by reference.

### BACKGROUND

### Technical Field

The disclosure relates to surgical instruments and, more particularly, to a hand actuated suction and irrigation device.

### Background of Related Art

In minimally invasive surgical procedures, including endoscopic and laparoscopic surgeries, a surgical access device permits the introduction of a variety of surgical instruments into a body cavity or opening. A surgical access device (e.g., a cannula) is introduced through an opening in tissue (i.e., a naturally occurring orifice or an incision) to provide access to an underlying surgical site in the body. The incision is typically made using an obturator having a blunt or sharp tip that has been inserted within the passageway of the surgical access device. For example, a cannula has a tube of rigid material with a thin wall construction, through which an obturator may be passed. The obturator is utilized to penetrate a body wall, such as an abdominal wall, or to introduce the surgical access device through the body wall, and is then removed to permit introduction of surgical instrumentation through the surgical access device to perform the surgical procedure.

In some surgeries, it is desirable to provide suction and/or irrigation at the surgical site. Accordingly, a simple device that selectively provides suction and/or irrigation while enabling the clinician to control the amount of suction and irrigation would be beneficial.

### SUMMARY

This disclosure describes a hand actuated suction and irrigation device that demonstrates a practical approach to meeting the performance requirements and overcoming usability challenges associated with supplying/removing fluids to and from a surgical site.

In accordance with the disclosure, a suction and irrigation device includes a handle assembly, an elongate shaft, and a fluid control assembly. The handle assembly includes a stationary handle and a movable handle pivotably secured to the stationary handle. The elongate shaft defines a lumen therethrough. The elongate shaft extends from the handle assembly. The fluid control assembly includes a first valve assembly having a first one-way valve, a pump interposed between the stationary and movable handles such that actuation of the movable handle causes compression of the pump, a tube extending between the first valve assembly and the pump, and a second valve assembly having a two-way valve assembly rotatable to establish selective communication between the pump and a supply line or a discharge line. When the first and second valve assemblies are in a suction mode, the pump is configured to receive a fluid from a surgical site when the movable handle is unactuated and the fluid in the pump is displaced towards the discharge line through the second valve assembly when the movable handle is actuated. When the first and second valve assemblies are in an irrigation mode, the pump is configured to receive a second fluid through the second valve assembly when the movable handle is unactuated and the second fluid in the pump enters the lumen of the elongate shaft through the first valve assembly when the movable handle is actuated.

In an aspect, the pump may include a bellows.

In another aspect, the first valve assembly may include a lever coupled to the first one-way valve to impart rotation to the first one-way valve to transition between the suction and irrigation modes.

In yet another aspect, the first one-way valve of the first valve assembly may be invertible to change the direction of the one-way valve.

In yet another aspect, when the first valve assembly is in the suction mode, the first one-way valve may direct the fluid into the tube through the first one-way valve.

In still yet another aspect, the second valve assembly may inhibit flow of a fluid therethrough when the second valve assembly is in the suction mode and the movable handle is unactuated.

In an aspect, the movable handle may define a camming slot, and the pump may have a camming pin cammingly engaging the camming slot.

In an aspect, when the second valve assembly is in the suction mode and the movable handle is actuated, the second valve assembly may provide communication between the pump and the discharge line connected to a collection cannister.

In another aspect, when the second valve assembly is in an irrigation mode and the movable handle is unactuated, the second valve assembly may provide communication between the pump and the supply line.

In yet another aspect, when the first valve assembly is in the irrigation mode and the movable handle is unactuated, the first valve assembly may block communication between the pump and the lumen of the elongate shaft.

In still yet another aspect, when the first valve assembly is in the irrigation mode and the movable handle is actuated, the first valve assembly may provide communication between the pump and the lumen of the elongate shaft.

In still yet another aspect, when the second valve assembly is in the irrigation mode and the movable handle is actuated, the second valve assembly may block communication between the pump and the supply line.

In accordance with another aspect of the disclosure, a suction and irrigation device includes a handle assembly including a stationary handle and a movable handle pivotably secured to the stationary handle, an elongate shaft defining a lumen therethrough, and a fluid control assembly. The elongate shaft extends from the handle assembly. The fluid control assembly includes a pump operatively supported on the handle assembly such that actuation of the movable handle causes compression of the pump, a first valve assembly providing selective communication between the pump and the elongate shaft, and a second valve assembly providing selective communication between the pump and a supply line or a discharge line. The first and second valve assemblies are transitionable between suction and irrigation modes. When the first and second valve assemblies are in the suction mode, the pump is configured to receive a fluid through the first valve assembly when the movable handle is unactuated and the fluid in the pump is displaced towards the discharge line through the second valve assembly when the handle is actuated. When the first and second valve assemblies are in the irrigation mode, the pump is configured to receive a second fluid through the second valve assembly when the movable handle is unactuated and the second fluid in the pump flows to the lumen of the elongate shaft through the first valve assembly when the movable handle is actuated.

In accordance with yet another aspect of the disclosure, a suction and irrigation device includes a handle assembly and a fluid control assembly including a pump coupled to the handle assembly such that actuation of the handle assembly causes compression of the pump and first and second valve assemblies transitionable between suction and irrigation modes. The pump has opposing first and second conduits. The first valve assembly is operatively coupled to the first conduit of the pump and the second valve assembly is operatively coupled to the second conduit of the pump. When the first and second valve assemblies are in the suction mode, the pump is configured to receive a fluid from a surgical site when the handle assembly is unactuated and the fluid in the pump is displaced towards the discharge line through the second valve assembly when the handle assembly is actuated. When the first and second valve assemblies are in the irrigation mode, the pump is configured to receive a second fluid through the second valve assembly when the handle assembly is unactuated and the second fluid in the pump flows into an elongate shaft extending from the handle assembly through the first valve assembly when the handle assembly is actuated.

In an aspect, the second fluid may be saline.

In another aspect, the fluid control assembly may further include a bifurcated tubing interposed between the second conduit of the pump and the second valve assembly.

### BRIEF DESCRIPTION OF THE DRAWINGS

Various aspects of the disclosure are described hereinbelow with reference to the drawings, which are incorporated and constitute a part of this specification, wherein:
FIG. 1 is a perspective view of a hand actuated suction and irrigation device in accordance with the disclosure;
FIG. 2 is a side view of the hand actuated suction and irrigation device of FIG. 1;
FIG. 3 is a top view of the hand actuated suction and irrigation device of FIG. 1;
FIG. 4 is a perspective view of the hand actuated suction and irrigation device of FIG. 1 taken along section line 4-4 of FIG. 1;
FIG. 5 is a perspective view of the hand actuated suction and irrigation device of FIG. 4, illustrating actuation of a handle assembly of the hand actuated suction and irrigation device;
FIG. 6 is a perspective view of a first valve assembly of a fluid control assembly of the hand actuated suction and irrigation device of FIG. 1;
FIG. 7 is a perspective view of a second valve assembly of the fluid control assembly of the hand actuated suction and irrigation device of FIG. 1;
FIGS. 8 and 9 are perspective views of a fluid control assembly of the hand actuated suction and irrigation device of FIG. 1, illustrating the fluid control assembly configured to provide suction; and
FIGS. 10 and 11 are perspective views of the fluid control assembly of FIGS. 8 and 9, illustrating the fluid control assembly configured to provide irrigation.

### DETAILED DESCRIPTION

The disclosed hand actuated suction and irrigation device is described in detail with reference to the drawings, in which like reference numerals designate identical or corresponding elements in each of the several views. As used herein, the term "distal," as is conventional, will refer to that portion of the instrument, apparatus, device, or component thereof which is farther from the user, while the term "proximal" will refer to that portion of the instrument, apparatus, device, or component thereof which is closer to the user. As used herein, the terms parallel and perpendicular are understood to include relative configurations that are substantially parallel and substantially perpendicular up to about + or - 10 degrees from true parallel and true perpendicular. In the following description, well-known functions or constructions are not described in detail to avoid obscuring the disclosure in unnecessary detail.

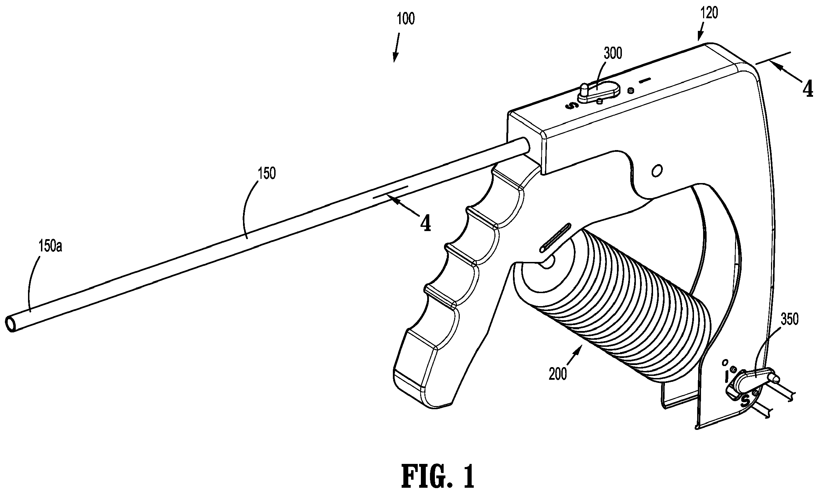

FIG. 1 illustrates a hand actuated suction and irrigation device in accordance with the disclosure is shown generally as a suction and irrigation device 100. The suction and irrigation device 100 enables the clinician to selectively provide suction or irrigation at a surgical site. In particular, the suction and irrigation device 100 enables the clinician to control the amount of suction or irrigation through selective actuation of an ergonomically designed handle assembly 120. The suction and irrigation device 100 provides single handed operation. The simple design of the suction and irrigation device 100 enables manual mechanical operation without any electrical components. In addition, the suction and irrigation device 100 provides continuous high-volume suction and irrigation compared to, e.g., when using a syringe.

The suction and irrigation device 100 includes a handle assembly 120, an elongate shaft 150 extending distally from the handle assembly 120, and a fluid control assembly 200 selectively controlling the flow of a fluid to and from the surgical site.

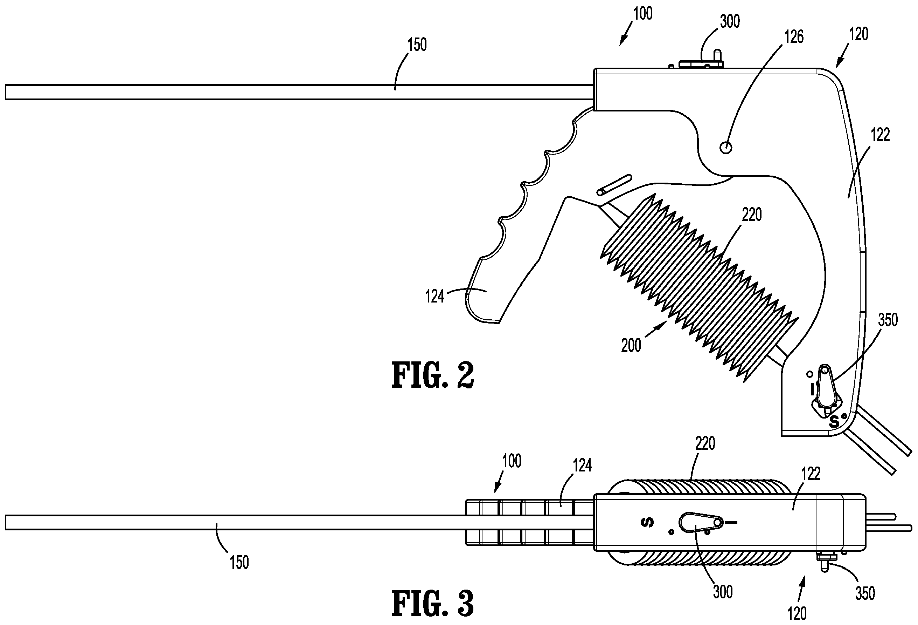

FIGS. 2 and 3 illustrate the handle assembly 120 including a stationary handle 122 and a movable handle 124 pivotably coupled to the stationary handle 122 about a pivot 126. The handle assembly 120 operably supports the fluid control assembly 200 to enable control of the amount of suction or irrigation by the clinician. In particular, the movable handle 124 is pivotably supported on the stationary handle 122 such that when the movable handle 124 is squeezed by the clinician, a pump 220 of the fluid control assembly 200 is compressed to provide suction or irrigation depending on the setting or mode selected by the clinician, as will be discussed below.

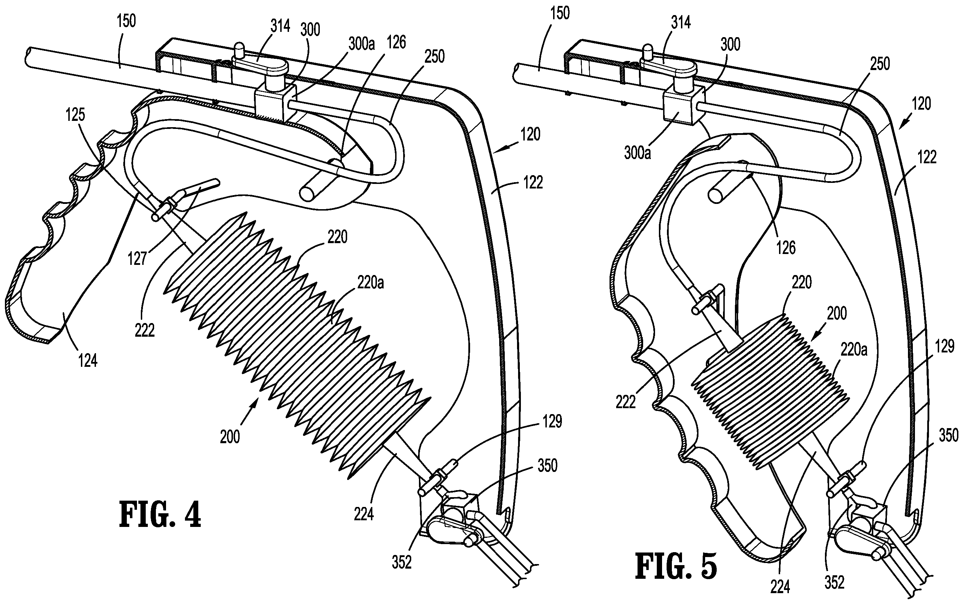

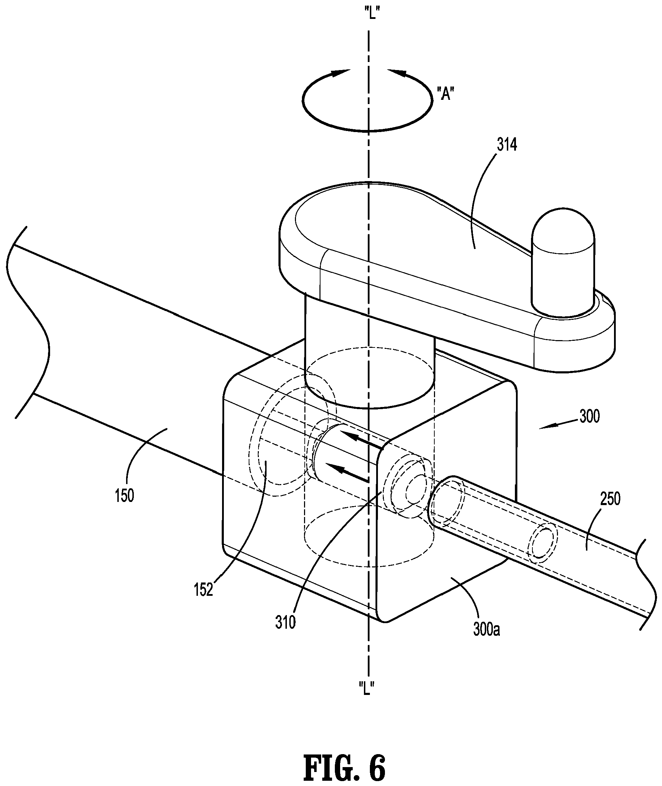

FIGS. 4 and 5 illustrate the fluid control assembly 200 operatively supported on the handle assembly 120. The fluid control assembly 200 includes a first valve assembly 300 in communication with a lumen 152 (FIG. 6) defined through the elongate shaft 150, a pump 220 in selective communication with the first valve assembly 300 via a tube 250, and a second valve assembly 350 in selective communication with the pump 220. In particular, FIG. 6 illustrates the first valve assembly 300 including a housing 300a, a one-way valve 310 (shown in phantom) disposed in the housing 300a, and a first lever 314 rotatably coupled to the one-way valve 310. For example, the one-way valve 310 may be a check valve. The first lever 314 is rotatable in the direction of an arrow "A" (about an axis "L-L") to invert the direction of the one-way valve 310. For example, when the first lever 314 is positioned for suction "S" (FIG. 1), the one-way valve 310 is oriented to enable a fluid to flow from the lumen 152 of the elongate shaft 150 to the tube 250 through the one-way valve 310. Inversely, when the first lever 314 is positioned for irrigation "I" (FIG. 1), the one-way valve 310 is oriented to enable a fluid to flow from the tube 250 to the lumen 152 of the elongate shaft 150 through the one-way valve 310.

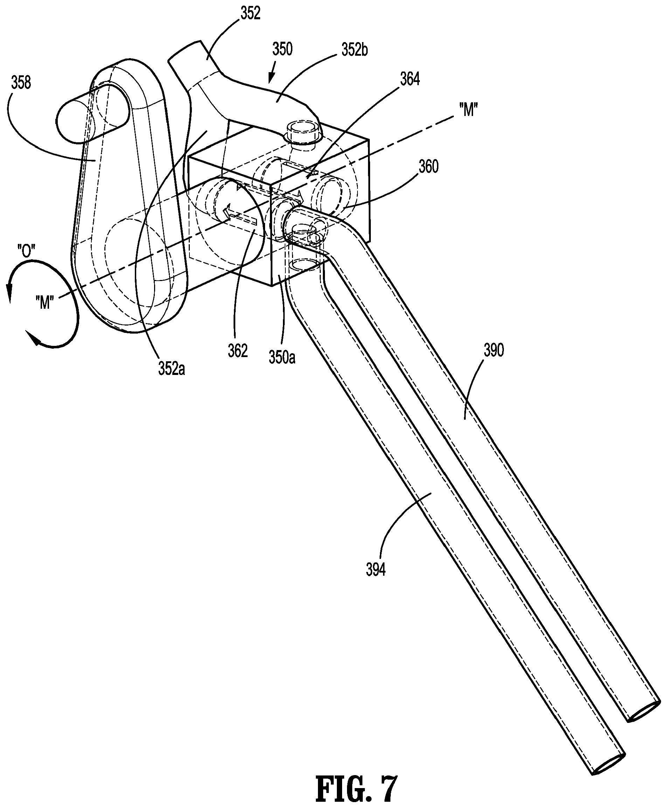

The pump 220 has a bellows 220a (FIG. 4) configured to retain a volume of a fluid therein and to displace a fluid through either ends of the pump 220. In particular, the pump 220 has first and second conduits 222, 224 on opposing ends thereof. The first conduit 222 is in communication with the tube 250. In addition, the first conduit 222 is coupled to a camming pin 125 configured to cammingly engage a camming slot 127 defined in the movable handle 124. Under such a configuration, when the movable handle 124 is squeezed by the clinician, the movable handle 124 pivots about the pivot 126 and the camming pin 125 rides along the camming slot 127 of the movable handle 124. In this manner, the bellows 220a of the pump 220 is compressed to selectively displace a fluid through one of the first or second conduits 222, 224. The second conduit 224 extends from the pump 220 towards the stationary handle 122. The second conduit 224 is secured to the stationary handle 122 by, e.g., a pin 129. In particular, the second conduit 224 is in selective communication with the second valve assembly 350. Further, the second conduit 224 is in communication with the bifurcated tubing 352 having an inlet tubing 352a (FIG. 7) and an outlet tubing 352b (FIG. 7).

FIG. 7 illustrates the second valve assembly 350 further including a housing 350a and a two-way valve assembly 360 disposed in the housing 350a. The two-way valve assembly 360 includes two one-way valves 362, 364 in selective communication with the respective inlet tubing 352a and the outlet tubing 352b. The one-way valves 362, 364 may be check valves. A second lever 358 selectively rotates the two-way valve assembly 360 about an axis "M-M" such that when the second lever 358 is positioned for irrigation "I" (FIG. 1), the one-way valve 362 places a supply line 390 in communication with the inlet tubing 352a. When the second lever 358 is positioned for suction "S" (FIG. 1), the one-way valve 364 is oriented to place the outlet tubing 352b in communication with a discharge line 394. The supply line 390 may be connected to a fluid supply to supply, e.g., saline, and the discharge line 394 may be connected to, e.g., a collection cannister, to collect fluids from the surgical site.

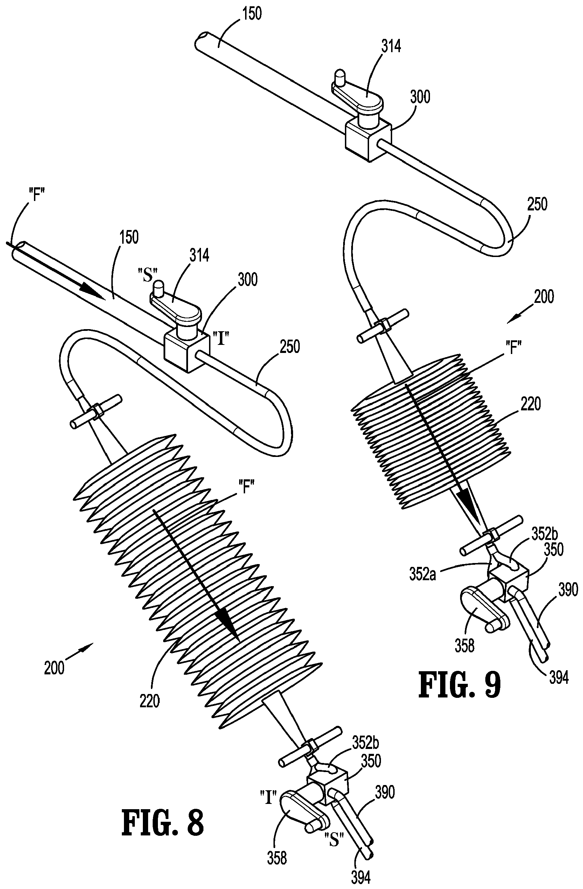

FIGS. 8 and 9 illustrate the fluid control assembly 200 in a suction mode "S". Specifically, the first and second levers 314, 358 of the first and second valve assemblies 300, 350 are placed in the respective suction setting "S". Such a configuration places the one-way valve 310 (FIG. 6) of the first valve assembly 300 in an orientation to enable fluid to flow from the lumen 152 (FIG. 6) of the elongate shaft 150 to the tube 250 while inhibiting backflow of the fluid into the lumen 152 of the elongate shaft 150. Fluids such as blood may flow through the first valve assembly 300 and into the pump 220 when the movable handle 124 (FIG. 2) is unactuated, i.e., the pump 220 is in the expanded state (FIG. 8). While the pump 220 is in the expanded state, the second valve assembly 350 inhibits flow of the fluid through the second valve assembly 350 such that the fluid is collected in the pump 220. When the clinician actuates the movable handle 124, i.e., the bellows 220a is compressed, the first valve assembly 300 inhibits the fluid in the pump 220 from flowing into the lumen 152 of the elongate shaft 150. The fluid flows to the outlet tubing 352b and out of the discharge line 394 through the second valve assembly 350 to remove fluids from the surgical site.

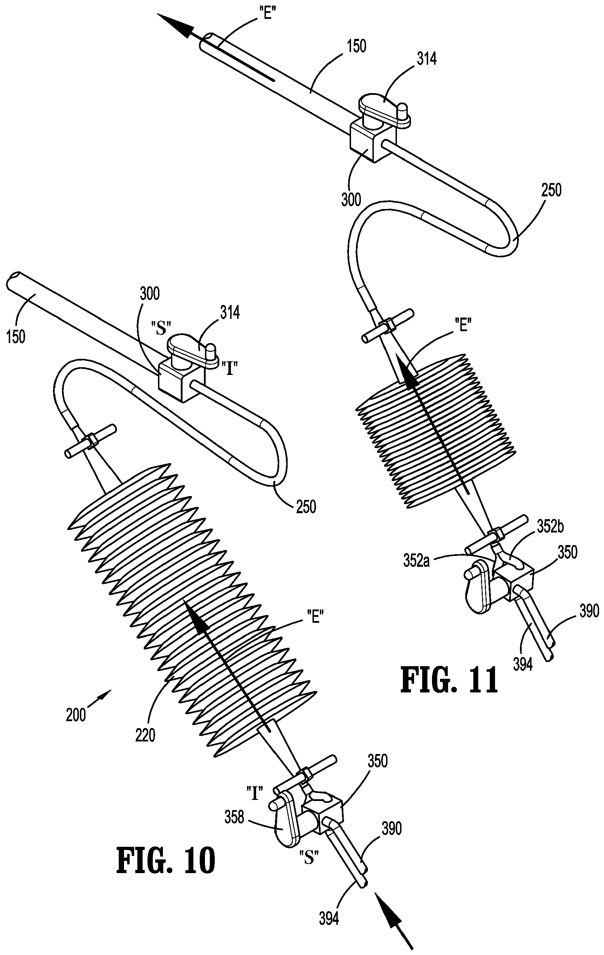

FIGS. 10 and 11 illustrate the fluid control assembly 200 in an irrigation mode "I". Specifically, the first and second levers 314, 358 of the first and second valve assemblies 300, 350 are placed in the respective irrigation setting "I". Such a setting places the two-way valve 350 to enable fluid such as, e.g., saline, to flow from the supply line 390 to the inlet tubing 352a and into the pump 220 in the expanded state (FIG. 10). While the pump 220 is in the expanded state, the first valve assembly 300 inhibits flow of the fluid through the first valve assembly 300 and into the lumen 152 of the elongate shaft 150 such that the fluid is collected in the pump 220. When the clinician actuates the movable handle 124, i.e., the bellows 220a is compressed, the second valve assembly 350 inhibits the fluid in the pump 220 from flowing into the supply line 390. The fluid flows out of the elongate shaft 150 through the first valve assembly 300 in order to supply the fluid such as, e.g., saline, to the surgical site.

In use, a distal end portion 150a (FIG. 1) of the elongate shaft 150 of the suction and irrigation device 100 is positioned within a patient (e.g., in the abdominal cavity). At this time, the body cavity may be inflated by supplying insufflation fluid to the body cavity through an inflation port. While a surgical procedure is being performed, the suction and irrigation device 100 may be utilized to apply suction to the surgical site to remove fluids therethrough or, alternatively, may provide fluids such as, e.g., saline, to the surgical site.

It will be understood that various modifications may be made to the disclosed suction and irrigation device 100. Therefore, the above description should not be construed as limiting, but merely as exemplifications of the disclosure. Those skilled in the art will envision other modifications within the scope and spirit of the disclosure.

The invention is described by the following numbered paragraphs:-
1. A suction and irrigation device comprising:
   a handle assembly including a stationary handle and a movable handle pivotably secured to the stationary handle;
   an elongate shaft defining a lumen therethrough, the elongate shaft extending from the handle assembly; and
   a fluid control assembly including:
      a first valve assembly having a first one-way valve;
      a pump interposed between the stationary and movable handles such that actuation of the movable handle causes compression of the pump;
      a tube extending between the first valve assembly and the pump; and
      a second valve assembly having a two-way valve assembly rotatable to establish selective communication between the pump and a supply line or a discharge line,
   wherein when the first and second valve assemblies are in a suction mode, the pump is configured to receive a fluid from a surgical site when the movable handle is unactuated and the fluid in the pump is displaced towards the discharge line through the second valve assembly when the movable handle is actuated,
   wherein when the first and second valve assemblies are in an irrigation mode, the pump is configured to receive a second fluid through the second valve assembly when the movable handle is unactuated and the second fluid in the pump enters the lumen of the elongate shaft through the first valve assembly when the movable handle is actuated.
2. The suction and irrigation device according to paragraph 1, wherein the pump includes a bellows.
3. The suction and irrigation device according to paragraph 1, wherein the first valve assembly includes a lever coupled to the first one-way valve to impart rotation to the first one-way valve to transition between the suction and irrigation modes.
4. The suction and irrigation device according to paragraph 1, wherein the first one-way valve of the first valve assembly is invertible to change the direction of the one-way valve.
5. The suction and irrigation device according to paragraph 1, wherein when the first valve assembly is in the suction mode, the first one-way valve directs the fluid into the tube through the first one-way valve.
6. The suction and irrigation device according to paragraph 1, wherein the second valve assembly inhibits flow of a fluid therethrough when the second valve assembly is in the suction mode and the movable handle is unactuated.
7. The suction and irrigation device according to paragraph 1, wherein the movable handle defines a camming slot, and the pump has a camming pin cammingly engaging the camming slot.
8. The suction and irrigation device according to paragraph 1, wherein when the second valve assembly is in the suction mode and the movable handle is actuated, the second valve assembly provides communication between the pump and the discharge line connected to a collection cannister.
9. The suction and irrigation device according to paragraph 1, wherein when the second valve assembly is in an irrigation mode and the movable handle is unactuated, the second valve assembly provides communication between the pump and the supply line.
10. The suction and irrigation device according to paragraph 1, wherein when the first valve assembly is in the irrigation mode and the movable handle is unactuated, the first valve assembly blocks communication between the pump and the lumen of the elongate shaft.
11. The suction and irrigation device according to paragraph 1, wherein when the first valve assembly is in the irrigation mode and the movable handle is actuated, the first valve assembly provides communication between the pump and the lumen of the elongate shaft.
12. The suction and irrigation device according to paragraph 1, wherein when the second valve assembly is in the irrigation mode and the movable handle is actuated, the second valve assembly blocks communication between the pump and the supply line.
13. A suction and irrigation device comprising:
   a handle assembly including a stationary handle and a movable handle pivotably secured to the stationary handle;
   an elongate shaft defining a lumen therethrough, the elongate shaft extending from the handle assembly; and
   a fluid control assembly including:
      a pump operatively supported on the handle assembly such that actuation of the movable handle causes compression of the pump;
      a first valve assembly providing selective communication between the pump and the elongate shaft; and
      a second valve assembly providing selective communication between the pump and a supply line or a discharge line, the first and second valve assemblies being transitionable between suction and irrigation modes,
   wherein when the first and second valve assemblies are in the suction mode, the pump is configured to receive a fluid through the first valve assembly when the movable handle is unactuated and the fluid in the pump is displaced towards the discharge line through the second valve assembly when the handle is actuated,
   wherein when the first and second valve assemblies are in the irrigation mode, the pump is configured to receive a second fluid through the second valve assembly when the movable handle is unactuated and the second fluid in the pump flows to the lumen of the elongate shaft through the first valve assembly when the movable handle is actuated.
14. The suction and irrigation device according to paragraph 13, wherein the pump has a bellows defining a volume to retain a fluid therein.
15. The suction and irrigation device according to paragraph 14, wherein actuation of the movable handle causes compression of the bellows.
16. The suction and irrigation device according to paragraph 13, wherein the pump is cammingly secured to the movable handle.
17. A suction and irrigation device comprising:
   a handle assembly; and
   a fluid control assembly including:
      a pump coupled to the handle assembly such that actuation of the handle assembly causes compression of the pump, the pump having opposing first and second conduits; and
      first and second valve assemblies transitionable between suction and irrigation modes, the first valve assembly operatively coupled to the first conduit of the pump and the second valve assembly operatively coupled to the second conduit of the pump;
   wherein when the first and second valve assemblies are in the suction mode, the pump is configured to receive a fluid from a surgical site when the handle assembly is unactuated and the fluid in the pump is displaced towards the discharge line through the second valve assembly when the handle assembly is actuated,
   wherein when the first and second valve assemblies are in the irrigation mode, the pump is configured to receive a second fluid through the second valve assembly when the handle assembly is unactuated and the second fluid in the pump flows into an elongate shaft extending from the handle assembly through the first valve assembly when the handle assembly is actuated.
18. The suction and irrigation device according to paragraph 17, wherein the second fluid is saline.
19. The suction and irrigation device according to paragraph 17, wherein the first valve assembly includes an invertible one-way valve for selective communication between the pump and the elongate shaft.
20. The suction and irrigation device according to paragraph 17, wherein the fluid control assembly further includes a bifurcated tubing interposed between the second conduit of the pump and the second valve assembly.

## Claims

1. A suction and irrigation device comprising:
a handle assembly including a stationary handle and a movable handle pivotably secured to the stationary handle;
an elongate shaft defining a lumen therethrough, the elongate shaft extending from the handle assembly; and
a fluid control assembly including:
a first valve assembly having a first one-way valve;
a pump interposed between the stationary and movable handles such that actuation of the movable handle causes compression of the pump;
a tube extending between the first valve assembly and the pump; and
a second valve assembly having a two-way valve assembly rotatable to establish selective communication between the pump and a supply line or a discharge line,
wherein when the first and second valve assemblies are in a suction mode, the pump is configured to receive a fluid from a surgical site when the movable handle is unactuated and the fluid in the pump is displaced towards the discharge line through the second valve assembly when the movable handle is actuated,
wherein when the first and second valve assemblies are in an irrigation mode, the pump is configured to receive a second fluid through the second valve assembly when the movable handle is unactuated and the second fluid in the pump enters the lumen of the elongate shaft through the first valve assembly when the movable handle is actuated.

2. The suction and irrigation device according to claim 1, wherein the pump includes a bellows.

3. The suction and irrigation device according to any preceding claim, wherein the first valve assembly includes a lever coupled to the first one-way valve to impart rotation to the first one-way valve to transition between the suction and irrigation modes; and/or wherein the first one-way valve of the first valve assembly is invertible to change the direction of the one-way valve.

4. The suction and irrigation device according to any preceding claim, wherein when the first valve assembly is in the suction mode, the first one-way valve directs the fluid into the tube through the first one-way valve.

5. The suction and irrigation device according to any preceding claim, wherein the second valve assembly inhibits flow of a fluid therethrough when the second valve assembly is in the suction mode and the movable handle is unactuated; and/or wherein the movable handle defines a camming slot, and the pump has a camming pin cammingly engaging the camming slot.

6. The suction and irrigation device according to any preceding claim, wherein when the second valve assembly is in the suction mode and the movable handle is actuated, the second valve assembly provides communication between the pump and the discharge line connected to a collection cannister; and/or wherein when the second valve assembly is in an irrigation mode and the movable handle is unactuated, the second valve assembly provides communication between the pump and the supply line.

7. The suction and irrigation device according to any preceding claim, wherein when the first valve assembly is in the irrigation mode and the movable handle is unactuated, the first valve assembly blocks communication between the pump and the lumen of the elongate shaft.

8. The suction and irrigation device according to any preceding claim, wherein when the first valve assembly is in the irrigation mode and the movable handle is actuated, the first valve assembly provides communication between the pump and the lumen of the elongate shaft; preferably wherein when the second valve assembly is in the irrigation mode and the movable handle is actuated, the second valve assembly blocks communication between the pump and the supply line.

9. A suction and irrigation device comprising:
a handle assembly including a stationary handle and a movable handle pivotably secured to the stationary handle;
an elongate shaft defining a lumen therethrough, the elongate shaft extending from the handle assembly; and
a fluid control assembly including:
a pump operatively supported on the handle assembly such that actuation of the movable handle causes compression of the pump;
a first valve assembly providing selective communication between the pump and the elongate shaft; and
a second valve assembly providing selective communication between the pump and a supply line or a discharge line, the first and second valve assemblies being transitionable between suction and irrigation modes,
wherein when the first and second valve assemblies are in the suction mode, the pump is configured to receive a fluid through the first valve assembly when the movable handle is unactuated and the fluid in the pump is displaced towards the discharge line through the second valve assembly when the handle is actuated,
wherein when the first and second valve assemblies are in the irrigation mode, the pump is configured to receive a second fluid through the second valve assembly when the movable handle is unactuated and the second fluid in the pump flows to the lumen of the elongate shaft through the first valve assembly when the movable handle is actuated.

10. The suction and irrigation device according to claim 9, wherein the pump has a bellows defining a volume to retain a fluid therein; and/or wherein actuation of the movable handle causes compression of the bellows.

11. The suction and irrigation device according to any of claims 9 to 10, wherein the pump is cammingly secured to the movable handle.

12. A suction and irrigation device comprising:
a handle assembly; and
a fluid control assembly including:
a pump coupled to the handle assembly such that actuation of the handle assembly causes compression of the pump, the pump having opposing first and second conduits; and
first and second valve assemblies transitionable between suction and irrigation modes, the first valve assembly operatively coupled to the first conduit of the pump and the second valve assembly operatively coupled to the second conduit of the pump;
wherein when the first and second valve assemblies are in the suction mode, the pump is configured to receive a fluid from a surgical site when the handle assembly is unactuated and the fluid in the pump is displaced towards the discharge line through the second valve assembly when the handle assembly is actuated,
wherein when the first and second valve assemblies are in the irrigation mode, the pump is configured to receive a second fluid through the second valve assembly when the handle assembly is unactuated and the second fluid in the pump flows into an elongate shaft extending from the handle assembly through the first valve assembly when the handle assembly is actuated.

13. The suction and irrigation device according to claim 12, wherein the second fluid is saline; and/or wherein the first valve assembly includes an invertible one-way valve for selective communication between the pump and the elongate shaft.

14. The suction and irrigation device according to claim 12 or claim 13, wherein the fluid control assembly further includes a bifurcated tubing interposed between the second conduit of the pump and the second valve assembly.
